# EUROPEAN PATENT APPLICATION

(11) **EP 0 824 930 A2**
(43) Date of publication of application: **25.02.1998**
(21) Application number: 97306280.5
(22) Date of filing: 19.08.1997
(51) Int. Cl.: A61M 25/00

(54) **Method for producing an insert molded catheter with securement windows**

(30) Priority: 20.08.1996 US 700003
(71) Applicant: JOHNSON & JOHNSON MEDICAL, INC., Arlington, Texas 76004-3030 (US)
(72) Inventor: Bogert, David L., Milton, West Virginia 25541 (US)
(74) Representative: Mercer, Christopher Paul

(57) **Abstract**

A method for producing a catheter assembly comprised of a catheter tube and a catheter hub assembled together in which a catheter tube is placed as a central insert into a mold for casting the catheter hub. The catheter tube is secured in place in the mold by at least one securing projection in the mold contacting and securing the catheter tube to prevent any movement or distortion thereof during the injection molding process. The catheter hub is then molded around the secured catheter tube insert in the mold, such that the resultant molded catheter assembly is molded with a securement window formed by each projection in the mold at which the catheter tube is exposed through the catheter hub. In one disclosed embodiment, the catheter tube is initially loaded onto a central mandrel positioned in the mold. The molding is performed with a two piece mold, wherein each mold piece defines a securing projection which contacts the catheter tube after the two piece mold is closed around the catheter tube on the mandrel. After the two piece mold is closed, a hollow cylindrical forming die is lowered onto the catheter tube positioned on the mandrel to deform the catheter tube into a flair. After the forming die is retracted, a top mold portion, which defines the end and interior of the catheter hub, is lowered onto the two piece mold, after which molten plastic is injected into the mold cavity.

## Description

### BACKGROUND OF THE INVENTION

### 1. Field of the Invention

The present invention relates generally to a method for producing an insert molded catheter with securement windows, and more particularly pertains to a method for producing an insert molded catheter with securement windows which uses an established robust technology, with established processes and equipment (i.e. a low tech approach), and also provides a low cost, low waste, high efficiency, approach.

Intravenous catheters are particularly used in medical applications for introducing blood, plasma, or other fluids into the circulatory, system of a patient. While IV catheters are available in several different types, one common type of catheter is constructed so as to be mounted upon a relatively long hollow cannula (needle) width a slight frictional fit. A hub is attached to one end of the catheter and is designed so as to be connectable with and detachable from an IV fluid supply line. To insert the catheter into a patient, the cannula and catheter together are inserted through the patient's skin into a vein, whereafter the cannula may be withdrawn, leaving the catheter in place therein.

### 2. Discussion of the Prior Art

It is highly desirable to provide a method for producing catheter assemblies, each comprising a catheter tube and a catheter hub assembled together. The prior art has attempted to commercially produce such catheter assemblies by methods involving insert molding of the catheter hub onto the catheter tube. Such an insert molding process places the catheter tube as an insert into the mold for the catheter hub, and the catheter hub is then molded around the catheter tube insert.

Upon initial consideration, insert molding of the catheter hub onto the catheter tube would seem to fulfill the above requirements. However, many companies which have attempted to use insert molding to produce catheters have found the process not to be sufficiently, reliable, and have returned to using eyelet assembly techniques, as are known in the prior art.

An examination of several prior art catheter designs which appeared to use insert molding revealed that none of them held the catheter tube tightly in position in the mold during molding of the plastic hub around the catheter tube. Since the plastic melt for the catheter hub is injected at high speeds and pressures(e.g. 10,000 psi), it appears that the relatively weak catheter tube would often be deformed by the injected plastic melt into configurations which would not robustly adhere to the injected plastic of the catheter hub.

### SUMMARY OF THE INVENTION

Accordingly, it is a primary object of the present invention to provide a method for producing an insert molded catheter with securement windows. '

A further object of the subject invention is the provision of a method for producing catheter assemblies, each comprising a catheter tube and a catheter hub assembled together, wherein the production method uses an established robust technology, with established processes and equipment (i.e. a low tech approach), and also provides a low cost, low waste, high efficiency approach.

The present invention uses an insert mold for the catheter hub having a "window" design, wherein the plastic catheter tube is securely retained in place during injection molding of the catheter hub by securing projections in the mold contacting and securing in place the plastic catheter tube, with each securing projection forming a window in the catheter hub assembly where the catheter tube is exposed in the final assembly. The steel mold with the securing projections which form the windows in the catheter hub holds the catheter tube firmly in place during the process of injection molding of the catheter hub, and prevents any distortions or movements of the plastic catheter tube.

This technical approach was verified by an inexpensive, relatively simple prototype using a low technology mold and molding machine. The resulting assembly had excellent adhesion between the catheter tube and the catheter hub, despite the inexpensive, low technology test approach of the prototype.

The present invention is particularly useful in a complete cell, which is a substantially automated production facility which starts with raw material to be molded and produces a finished packaged at its output, particularly a complete cell which uses inline molding to produce low cost catheter products with minimal technical support.

In accordance with the teachings herein, the present invention provides a method for producing a catheter assembly comprised of a catheter tube and a catheter hub assembled together in which a catheter tube is placed as a central insert into a mold for casting the catheter hub. Pursuant to the present invention, the catheter tube is secured in place in the mold by at least one securing projection in the mold contacting and securing the catheter tube to prevent any movement or distortion thereof during the injection molding process. The catheter hub is then molded around the secured catheter tube insert in the mold, such that the resultant molded catheter assembly is molded with a securement window formed by each projection in the mold at which the catheter tube is exposed through the catheter hub.

In greater detail, the catheter tube is initially loaded onto a central mandrel positioned in the mold, and then the catheter tube is secured in place in the mold by two securing projections contacting and securing the catheter tube. The molding is performed with a two piece mold, wherein each mold piece defines a securing projection which contacts the catheter tube. The catheter tube is initially loaded onto the mandrel in the mold, and then the two piece mold is closed around the catheter tube on the mandrel. After the two piece mold is closed, a hollow cylindrical forming die is lowered onto the catheter tube positioned on the mandrel to deform the catheter tube into a flair. After the forming die is retracted, a top mold portion, which defines the end and interior of the catheter hub, is lowered onto the two piece mold, after which molten plastic is injected into the mold cavity.

In one disclosed embodiment, first and second two piece molds are positioned on a conveyer, and a top mold portion, which defines the end and interior of the catheter hub, is lowered in turn onto each two piece mold, after which molten plastic is injected into the mold cavity. In operation, the first two piece mold is prepared for an injection molding operation while the second two piece mold is being used with the top mold portion in an injection molding operation. The second two piece mold is then unloaded and prepared for a subsequent injection molding operation while the first two piece mold is being used with the top mold portion in an injection molding operation.

### BRIEF DESCRIPTION OF THE DRAWINGS

The foregoing objects and advantages of the present invention for a method for producing an insert molded catheter with securement windows may be more readily understood by one skilled in the art with reference being had to the following detailed description of several preferred embodiments thereof, taken in conjunction with the accompanying drawings wherein like elements are designated by identical reference numerals throughout the several views, and in which:
Figure 1 is a perspective view of an insert molded catheter with securement windows constructed pursuant to the teachings of the present invention, and illustrates one of two securement windows provided in the insert molded catheter;
Figure 2 is an end elevational view of the insert molded catheter with securement windows shown in Figure 1 and constructed pursuant to the teachings of the present invention;
Figure 3 is a longitudinal sectional view of the insert molded catheter with securement windows of Figures 1 and 2, taken along the direction of arrows 3-3 in Figure 2;
Figure 4 is a longitudinal sectional view of the insert molded catheter with securement windows of Figures 1 and 2, taken along the direction of arrows 4-4 in Figure 2;
Figure 5 is a cross sectional view of the insert molded catheter with securement windows of Figures 1 and 2, taken along the direction of arrows 5-5 in Figure 3;
Figures 6, 7 and 8 illustrate a first step in a method for producing an insert molded catheter with securement windows pursuant to the present invention in which a blank catheter tube is loaded onto a mandrel in a mold, wherein Figure 6 is a front elevational view of the molding apparatus during the first step, Figure 7 is an enlarged top plan view taken along arrows 7-7 in Figure 6, and Figure 8 is an enlarged elevational sectional view of Figure 7;

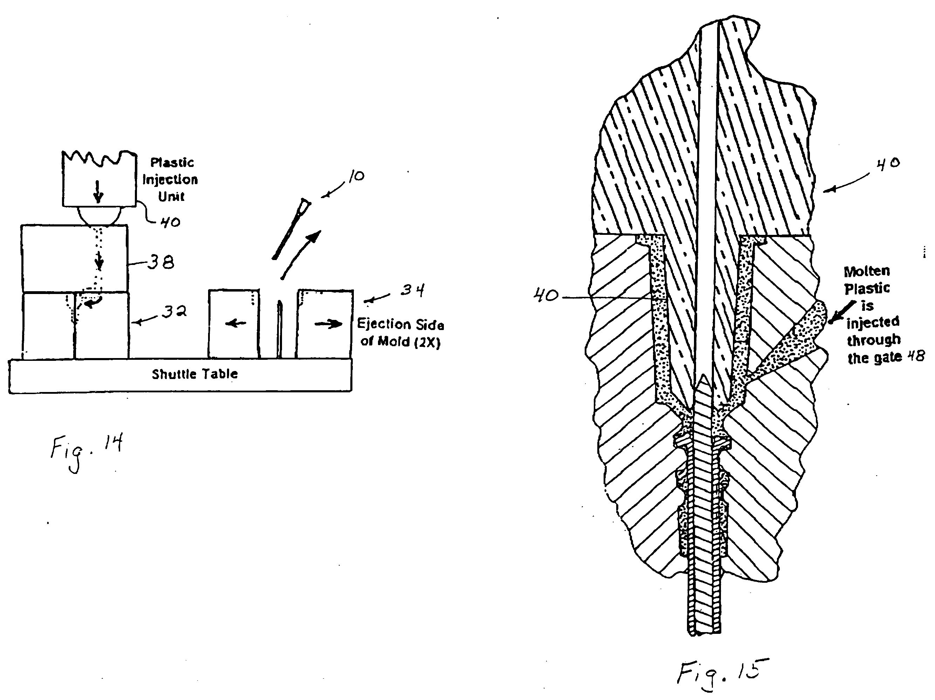

Figures 9, 10 and 11 illustrate second and third steps in a method for producing an insert molded catheter with securement windows pursuant to the present invention in which in the second step the two halves of the mold are closed together, securely holding the blank catheter tube in place therein, and in the third step, a hot forming die is lowered down onto the mandrel and forms the catheter tube into a flair, wherein Figure 9 is a front elevational view of the molding apparatus during the second and third steps, Figure 10 is an enlarged top plan view taken along arrows 10-10 in Figure 9, and Figure 11 is an enlarged elevational sectional view of the apparatus of Figure 10;
Figures 12 and 13 illustrate fourth and filth steps in a method for producing an insert molded catheter with securement windows pursuant to the present invention in which in the fourth step , the top portion of the mold and the plastic injection unit lift off one of the ejector sides of the molding apparatus (this side containing a completed hub and catheter tube), and in the fifth step, the shuttle table indexes over so that the other ejector side of the mold is under the top portion of the mold, wherein Figure 12 is a front elevational view of the molding apparatus during the fourth and fifth steps, and Figure 13 is an enlarged elevational sectional view through the left unfilled mold side of the molding apparatus of Figure 12; and Figures 14 and 15 illustrate sixth and seventh steps in a method for producing an insert molded catheter with securement windows pursuant to the present invention in which in the sixth step, the top portion of the mold and the plastic inspection unit are lowered onto the left unfilled mold side and molten plastic is injected into the hub cavity, surrounding and capturing the supported and formed end of the catheter tube, and in the seventh step, the right ejector mold side opens and a molded catheter assembly is removed therefrom.

### DETAILED DESCRIPTION OF THE DRAWINGS

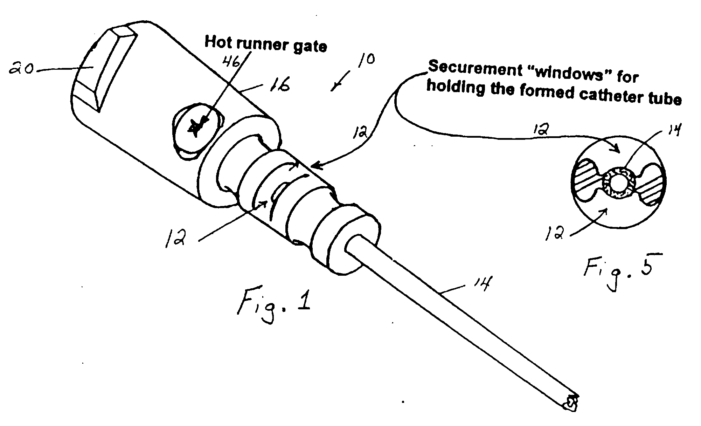

Referring to the drawings in detail, Figure 1 is a front perspective view of an insert molded catheter assembly 10 with securement windows constructed pursuant to the teachings of the present invention, and illustrates one of two securement windows 12 provided in the insert molded catheter assembly 10. The catheter assembly comprises a catheter tube 14, typically formed of polyurethane or Teflon®, and a catheter hub 16, typically formed of polypropylene or polyethylene.

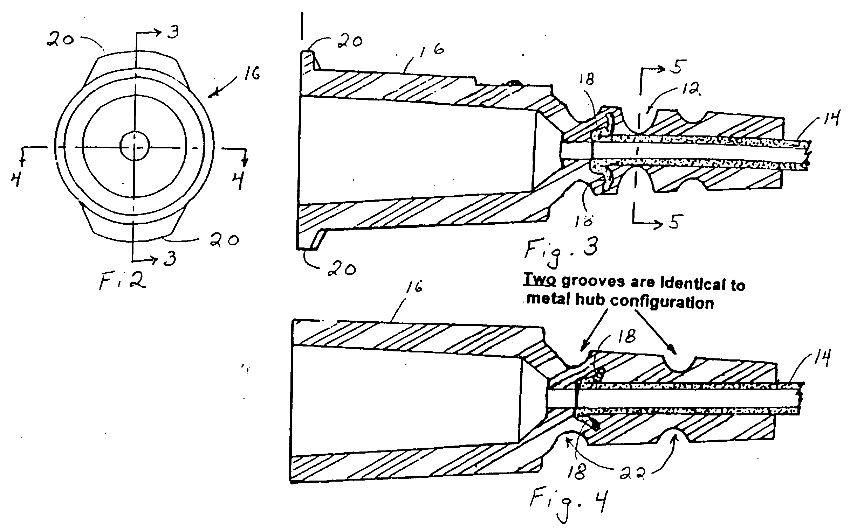

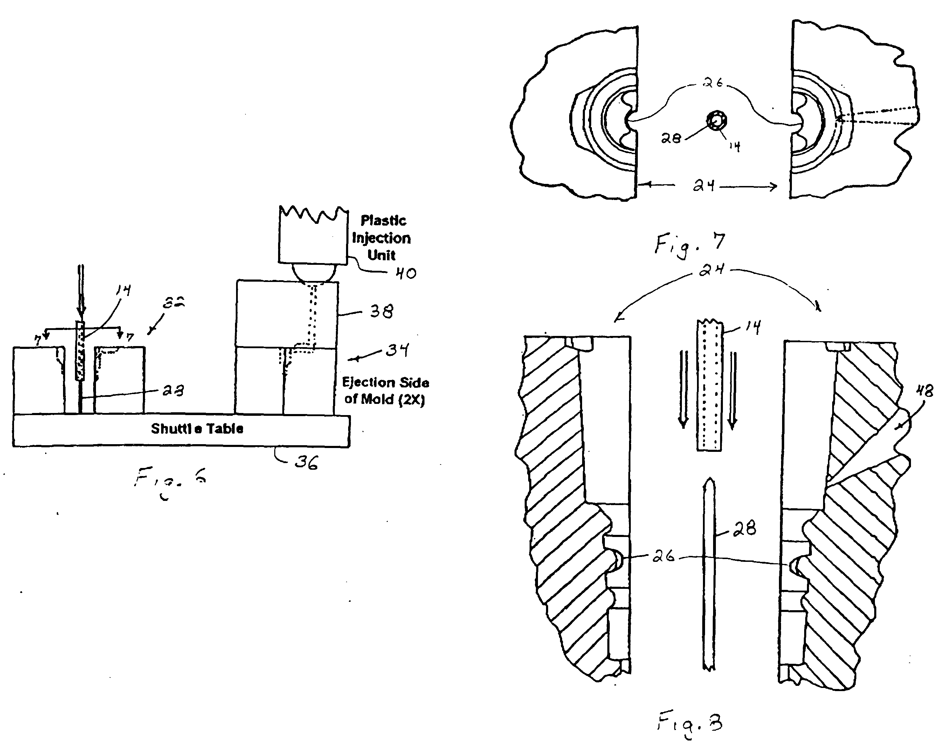

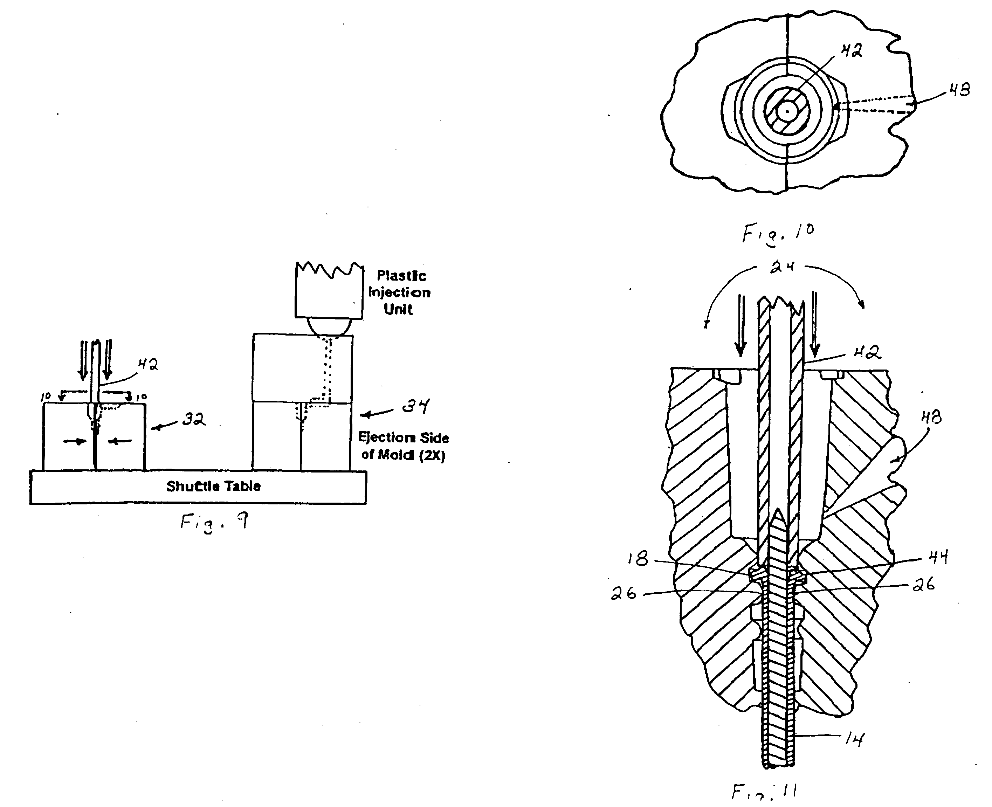

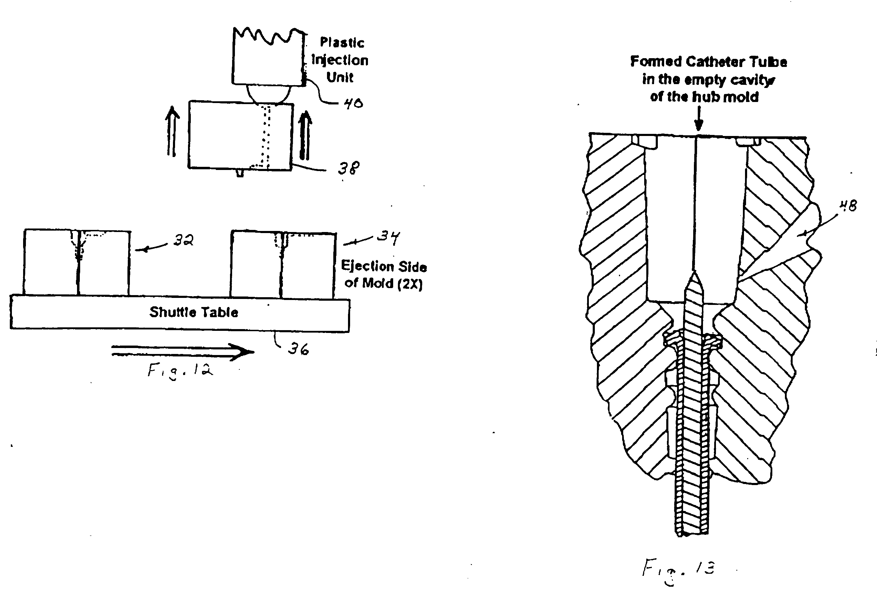

Figure 2 is an end elevational view of the insert molded catheter with securement windows 10 shown in Figure 1, while Figures 3 and 4 are respectively longitudinal sectional views of the insert molded catheter 10 of Figure 1, taken along the direction of respectively arrows 3-3 and 4-4 in Figure 2, and Figure 5 is a cross sectional view of the insert mold catheter taken along the direction of arrows 5-5 in Figure 3.

The present invention produces the catheter assembly 10 by insert molding of the catheter hub 16 onto and around the catheter tube 14. Such an insert molding process places the catheter tube 14 as an insert into the mold for casting the catheter hub 16, and the catheter hub 16 is then molded around the catheter tube 14 insert. The catheter tube 14 is typically initially formed by extrusion, after which it is cut into desired lengths. The extruded and cut catheter tube is then deformed or swaged at one end to form a plug 18 to promote adherence and securement of the catheter tube 14 to and in the catheter hub 16 which is subsequently molded therearound.

The catheter hub 16 is typically formed with Luer lugs 20 used to secure the catheter, as is well known in the art. The catheter hub is formed with two longitudinally spaced grooves 22 therearound, which physicians sometimes use to secure the catheter assembly to a patient by suture string wrapped around a groove 22.

The present invention uses an insert mold for casting the catheter hub having a window design, wherein the plastic catheter tube 14 is securely retained in place therein during injection molding of the hub 16 by securing projections in the mold contacting the plastic catheter tube 14. Each securing projection forms a resultant window 12 in the catheter hub 16 where the catheter tube 14 is exposed in the final catheter assembly.

As illustrated in Figures 8 and 11, a two piece steel mold 24 is formed with securing projections 26 exactly in the shape of the windows 12 which hold the catheter tube 14 firmly in placing during the process of injection molding of the catheter hub 16 around the catheter tube 14. The securing projections 26 prevent any distortions of the plastic catheter tube caused by the high pressure (e.g. 10,000 psi) and temperature injection molding process. A preferred number of securing projections in the hub mold is two, producing a catheter assembly 10 with two securement windows 12 as illustrated in Figures 1 and 5, although other embodiments can use from one to five or more securing projections.

Figures 6, 7 and 8 illustrate a first step in a method for producing an insert molded catheter with securement windows pursuant to the present prevention in which a blank precut catheter tube 14 is loaded onto a tapered mandrel 28 in the mold 24. Figure 6 is a front elevational view of the molding apparatus during the first step, while Figure 7 is an enlarged top plan view taken along arrows 7-7 in Figure 6, and Figure 8 is an enlarged elevational sectional view of Figure 7.

The molding apparatus 30 includes first and second two piece molds 32 and 34 arranged on a shuttle table 36. The first and second two piece molds 32 and 34 are alternately conveyed by the shuttle table 36 to a position beneath a top mold section 38 and plastic injection unit 40. In operation, one two piece mold is prepared for an injection molding operation as illustrated in the steps in Figures 6-15 while the other two piece mold is being used in an injection molding operation. The other two piece mold is then unloaded and prepared for a subsequent injection molding operation while the first two piece mold is being used in an injection molding operation, all as illustrated in Figure 6-15. Figures 6-8 illustrate a catheter tube 14 being loaded onto a mandrel 28 in the first two piece mold 32, while the second two piece mold 34 is being employed in an injection molding operation.

Figures 9, 10, and 11 illustrate second and third steps in a method for producing an insert molded catheter with securement windows pursuant to the present invention. During the second step the two halves of the mold 32 are closed together, with the projections 26 securely holding the blank catheter tube in place therein. In the third step a hot forming die 42 is lowered down onto the mandrel 28 and catheter tube and deforms the catheter tube into the flair 18 in an annular depression 44 in the mold. Figure 9 is a front elevational view of the molding apparatus during the second and third steps, while Figure 10 is an enlarged top plan view taken along arrows 10-10 in Figure 9, and Figure 11 is an enlarged elevational sectional view of Figure 10.

Figures 12 and 13 illustrate fourth and fifth steps in a method for producing an insert molded catheter with securement windows pursuant to the present invention. During the fourth step the top portion of the mold 38 and the plastic injection unit 40 lift off of the mold 34 (this half now contains a completed hub and catheter tube assembly). In the fifth step, the shuttle table 36 indexes over so that the first mold 32 is under the top portion of the mold. Figure 12 is a front elevational view of the molding apparatus during the fourth and fifth steps, while Figure 13 is an enlarged elevational sectional view through the molding apparatus.

Figures 14 and 15 illustrate sixth and seventh steps in a method for producing an insert molded catheter with securement windows pursuant to the present invention. During the sixth step, the top portion of the mold 38 and the plastic injection unit 40 are lowered onto the unfilled mold 32 and molten plastic is injected into the hub cavity, surrounding and capturing the supported and formed end of the catheter tube therein. In the seventh step the second ejection mold side 44 is opened and a molded catheter assembly 10 is removed therefrom.

The catheter tube still needs to have a bevel placed on the dull distal end, away from the catheter hub. Catheters have distal ends which are those ends to be inserted through the skin of a patient. The use of a catheter with a blunt distal or leading end is not desirable since the blunt tip tends to resist insertion into the skin, thereby increasing the difficulty and trauma of insertion. Moreover, insertion of a catheter with a blunt tip increases the irritation to the surrounding tissue, and perhaps more importantly, adds significantly to the pain and discomfort of the patient during insertion. Consequently the distal tip on IV catheter 18 is subsequently beveled to eliminate the aforementioned problems.

A preferred molding arrangement can use a multicavity cammed two piece mold, although other types of molding arrangements can also be used in alternative embodiments. The molding arrangement produces a minor depression 46 at the location of the hot runner gate 48.

The present invention provides a method for producing catheter assemblies which uses an established robust technology, with established processes and equipment, and provides a low cost, low waste, high efficiency approach for commercially, producing catheter assemblies. The present invention is particularly useful in a complete cell which uses in-line molding to produce low cost catheter products with minimal technical support.

While several embodiments and variations of the present invention for a method for producing an insert molded catheter with securement windows are described in detail herein, it should be apparent that the disclosure and teachings of the present invention will suggest many alternative designs to those skilled in the art.

## Claims

1. A method for producing a catheter assembly comprised of a catheter tube and a catheter hub assembled together, comprising:
a. placing a catheter tube as a central insert into a mold for casting the catheter hub;
b. securing the catheter tube in place in the mold during the injection molding process to prevent any movement or distortion of the catheter tube in the mold by at least one securing projection in the mold contacting and securing the catheter tube during the injection molding process; and
c. molding the catheter hub around the secured catheter tube insert in the mold.

2. A method for producing a catheter assembly as claimed in claim 1, wherein the resultant molded catheter assembly is molded with a securement window formed by each projection in the mold at which the catheter tube is exposed through the catheter hub.

3. A method for producing a catheter assembly as claimed in claim 1, including securing the catheter tube in place in the mold by two securing projections contacting and securing the catheter tube from two opposite directions.

4. A method for producing a catheter assembly as claimed in claim 1, wherein the catheter tube is loaded onto a central mandrel positioned in the mold.

5. A method for producing a catheter assembly as in claim 1, wherein the molding is performed with a two piece mold, wherein each mold piece defines a securing projection which contacts the catheter tube.

6. A method for producing a catheter assembly as in claim 5, wherein the catheter tube is initially loaded onto a mandrel in the mold, and the two piece mold is then closed around the catheter tube on the mandrel.

7. A method for producing a catheter assembly as in claim 5, wherein, after the two piece mold is closed, a hollow cylindrical forming die is lowered onto the catheter tube positioned on the mandrel to deform the catheter tube into a flair.

8. A method for producing a catheter assembly as in claim 7, wherein after the forming die is retracted, a mold top mold portion, which defines the end and interior of the catheter hub, is lowered onto the two piece mold, after which molten plastic is injected into the mold cavity.

9. A method for producing a catheter assembly as in claim 5, further including a top mold portion, which defines the end and interior of the catheter hub, which is lowered onto the two piece mold, after which molten plastic is injected into a mold cavity defined between the two piece mold and the top mold portion.

10. A method for producing a catheter assembly as in claim 5, further including first and second two piece molds positioned on a conveyer, and further including a top mold portion, which defines the end and interior of the catheter hub, which is lowered onto each two piece mold, after which molten plastic is injected into a mold cavity defined between the two piece mold and the top portion, and wherein in operation, the first two piece mold is prepared for an injection molding operation while the second two piece mold is being used with the top mold portion in an injection molding operation, and then the second two piece mold is unloaded and prepared for a subsequent injection molding operation while the first two piece mold is being used with the top mold portion in an injection molding operation.
